# EUROPÄISCHE PATENTSCHRIFT

(11) **EP 0 865 278 B1**
(45) Veröffentlichungstag und Bekanntmachung des Hinweises auf die Patenterteilung: **15.05.2002**
(21) Anmeldenummer: 96939875.9
(22) Anmeldetag: 21.11.1996
(51) Int. Cl.: A61K 31/44, A61P 31/10

(54) **VERWENDUNG VON 1-HYDROXY-2-PYRIDONEN ZUR BEHANDLUNG VON SCHWER THERAPIERBAREN SCHLEIMHAUTERKRANKUNGEN**
USE OF 1-HYDROXY-2-PYRIDONES FOR TREATING MUCOSA DISEASES WHICH ARE DIFFICULT TO TREAT
UTILISATION DE 1-HYDROXY-2-PYRIDONES POUR TRAITER DES MALADIES DIFFICILES A TRAITER AFFECTANT LES MUQUEUSES,

(30) Priorität: 04.12.1995 DE 19545139; 30.10.1996 DE 19643831
(43) Veröffentlichungstag der Anmeldung: 23.09.1998
(73) Patentinhaber: Aventis Pharma Deutschland GmbH, 65929 Frankfurt am Main (DE)
(72) Erfinder: KRAEMER, Karl, Theodor, D-63225 Langen (DE); BOHN, Manfred, D-65719 Hofheim (DE)
(86) Internationale Anmeldenummer: EP9605132
(87) Internationale Veröffentlichungsnummer: WO9720560

(56) Entgegenhaltungen:
- EP-A- 0 241 918
- US-A- 3 968 118
- MYCOSES, Bd. 33, Nr. 4, 1990, BERLIN, Seiten 191-202, XP000651618 RAETHER ET AL: "Rilopisox - a New Hydroxypyridone Antifungal with Fungicidal Properties"
- ANNALS NEW YORK ACADEMY OF SCIENCES, Bd. 544, 1988, NEW YORK, Seiten 329-337, XP000651698 HAENEL ET AL: "Evaluation of Fungicidal Action in vitro and in a Skin Model"
- ARZNEIMITTEL-FORSCHUNG, Bd. 45, Nr. 1, 1995, GERMANY, Seiten 84-87, XP002027401 BRAGA ET AL: "Inhibition of Candida Albicans Adhesiveness to Human Buccal and Vaginal cells by Sub-inhibitory Concentration of Rilopirox"
- REX ET AL: 'Resistance to Candida Species to Fluconazole' ANTIMICROBIOAL AGENTS AND CHEMOTHERAPY Bd. 39, Januar 1995, Seiten 1 - 8

## Beschreibung

Die Anzahl schwer therapierbarer Schleimhauterkrankungen hat in neuerer Zeit mit immer noch steigender Tendenz erheblich zugenommen. Zu den schwer therapierbaren Schleimhauterkrankungen zählen heute in erster Linie Candidosen im Bereich der Mund- und Vaginalschleimhaut.

Unter Candidosen versteht man Infektionen, die meist durch Candida albicans, aber auch durch eine Vielzahl anderer, opportunistisch wachsender Candidaspezies (C. krusei, C. tropicalis, Candida glabrata u.v.a.) hervorgerufen werden. Die ohnehin in der Mundhöhle, im Magendarm sowie in der Vagina oft vorhandenen Hefepilze vermehren sich unter besonderen Bedingungen und nehmen parasitär-pathogenen Charakter an. Hefepilze vermögen unter Umständen Haut und ihre Anhangsgebilde, sämtliche hautnahen Schleimhäute sowie mehrere innere Organe (Ösophagus, Lunge u.a.) zu besiedeln und dabei ein bemerkenswertes breites Spektrum von Erkrankungen hervorzurufen.

Insbesondere Schwangerschafter, Stoffwechselerkrankungen, Infektionskrankheiten, Tumoren und Immundefekte vermögen das Auftreten einer Candidose zu begünstigen. Als lokal begünstigende Faktoren gelten mechanische Reizung (z.B. Zahnprothesen), Okklusion, Feuchtigkeit bzw. feuchte Wärme.

Das Auftreten einer ausgedehnten oralen Candidose gilt heute in den meisten Ländern als eines der wichtigsten klinischen Zeichen einer gestörten Immunfunktion. Persistierende orale Candidosen markieren bei vielen HIV-Patienten den Übergang zur Immundekompensation. Bei fortgeschrittener Immundefizienz treten auch erosive, zum Teil auch ulzerierende Entzündungen unter Einbeziehung der Gingiva auf, nicht selten sind Candidabalanitis, Candidavulvitis und candidabedingte Analekzeme. Intestinale Infektionen und Candidasepsis werden ebenfalls beobachtet.

Bei immunkompetenten Kranken gilt Nystatin als Mittel der Wahl zur Lokaltherapie von Candida-Infektionen, doch bei HIV-infizierten, immundefizienten Patienten zeigt die klinische Erfahrung, daß die Nystatin Therapie allein häufig nicht ausreicht. In diesen Fällen hat sich die systemische Therapie mit Antimykotika vom Azoltyp durchgesetzt. Candida Stämme mit Azolresistenz waren bis zum Jahre 1989 nahezu unbekannt. Durch das Auftreten von Mischinfektionen mit den Protozoen Stämmen Trichomonas vaginalis und Entamoeba histolytica erwies sich jedoch die Behandlung von Vaginalcandidosen häufig als schwierig.

Seit der Verwendung von Fluconazol zur Rezidivprophylaxe von oropharyngealen Candidosen bei HIV-Patienten ist jedoch die Anzahl der bekannt gewordenen Azolresistenzen dramatisch angestiegen. In der Fachliteratur wurde bis einschließlich 1. Halbjahr 1995 in 98 Publikationen über die Azolresistenz von Candida Stämmen berichtet.

In den Dokumenten EP 0 241 918 oder US 4 797 409 werden die Herstellung von 1-Hydroxy-2-pyridonen und ihre Anwendung zur Bekämpfung hauptsächlich von Infektionen durch Pilze und Hefe sowie Arzneimittel, die diese Verbindungen enthalten, beschrieben.

Aufgabe der vorliegenden Erfindung ist daher die Bereitstellung von topischen Arzneimittelzubereitungen, die dazu geeignet sind, vorhandene intrinsische und erworbene fluconazolresistenzen von Candida Stämmen zu durchbrechen.

Es wurde nun gefunden, daß sich Verbindungen der Formel ! hervorragend zur Behandlung von Candidosen, die durch Hefestämme mit intrinsischer und erworbener fluconazolresistenz verursacht werden, eignen. Außerdem zeichnen sich die genannten Verbindungen durch ihre für Therapiezwecke ausreichende Aktivität gegenüber den Problemkeimen Trichomonas vaginalis und Entamoeba histolytica, die häufig Ursache für das Entstehen von Mischinfektionen bei Vaginatcandidosen sind, aus.

Die Erfindung betrifft die Verwendung der Verbindung der Formel I 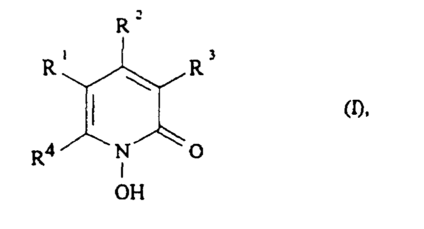 worin R¹, R² und R³, die gleich oder verschieden sind, Wasserstoffatom oder Alkyl mit 1-4 Kohlenstoffatomen bedeuten, und R⁴ einen gesättigten Kohlenwasserstoffrest mit 6 bis 9 Kohlenstoffatomen oder einen Rest der Formel II bedeutet 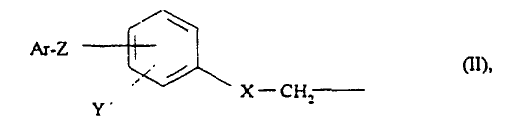 wobei
- X: S oder O bedeutet,
- Y: Wasserstoffatom oder bis zu 2 Halogenatome wie Chlor und/oder Brom bedeutet,
- Z: eine Einfachbindung oder die zweiwertigen Reste O, S, -CR₂-, (R = H oder C₁-C₄-Alkyl) oder andere zweiwertige Reste mit 2-10 kettenförmig verknüpften C- und gegebenenfalls O- und/oder S-Atomen, wobei - wenn die Reste 2 oder mehr O- und/oder S-Atome enthalten - letztere durch mindestens 2 C-Atome voneinander getrennt sein müssen und wobei 2 benachbarte C-Atome auch durch eine Doppelbindung miteinander verknüpft sein können und die freien Valenzen der C-Atome durch H und/oder C₁-C₄ Alkylgruppen abgesättigt sind, bedeutet,
- Ar: ein aromatisches Ringsystem mit bis zu zwei Ringen, das durch bis zu drei Reste aus der Gruppe Fluor, Chlor, Brom, Methoxy, C₁-C₄-Alkyl, Trifluormethyl und Trifluormethoxy substituiert sein kann, bedeutet,
zur Herstellung eines Arzneimittels zur topischen Behandlung von Pilzerkrankungen, die durch fluconazol-resistente Pilze verursacht werden.

In den Resten "Z" sind die C-Kettenglieder vorzugsweise CH₂-Gruppen. Wenn die CH₂-Gruppen durch C₁-C₄ Alkylgruppen substituiert sind, sind CH₃ und C₂H₅ bevorzugte Substituenten.

Beispielhafte Reste "Z" sind:
-O-, -S-, -CH₂-, -(CH₂)ₘ-(m=2-10), -C(CH₃)₂-, -CH₂O-, -OCH₂-, -CH₂S-, -S CH₂-, - S CH (C₂H₅)-, -CH=CH-CH₂O-, -O-CH₂-CH=CH-CH₂O-, -O CH₂-CH₂O-, -O CH₂-CH₂CH₂O-, -S CH₂ CH₂ CH₂ S-, - S CH₂ CH₂ CH₂ CH₂ O -, -S CH₂ CH₂ O CH₂ CH₂ O-, -S CH₂ CH₂ 0 CH₂ CH₂ O-CH₂ CH₂ S-, oder -S-CH₂-C (CH₃)₂-CH₂-S-.

Der Rest "S" bedeutet Schwefelatom, der Rest "O" bedeutet Sauerstoffatom. Der Begriff "Ar" bedeutet Phenyl und kondensierte Systeme wie Naphthyl, Tetrahydronaphthyl und Indenyl, sowie isolierte Systeme wie solche, die sich vom Biphenyl, Diphenylalkanen, Diphenylethern und Diphenylthioethem ableiten.

Wichtige Vertreter der durch die Formel I charakterisierten Verbindungsklasse sind:
6-[4-(4-Chlor-phenoxy)-phenoxymethyl)-1-hydroxy-4-methyl-2-pyridon;
6-[4-(2,4-Dichlor-phenoxy)-phenoxymethyl]-1-hydroxy-4-methyl-2-pyridon;
6-(Biphenylyl-4-oxy-methyl)-1-hydroxy-4-methyl-2-pyridon;
6-(4-Benzyl-phenoxymethyl)-1-hydroxy-4-methyl-2-pyridon;
6-[4-(2,4-Dichlorbenzyloxy)-phenoxy-methyl]-1-hydroxy-4-methyl-2-pyridon;
6-[4-(4-Chlor-phenoxy)-phenoxymethyl]-1-hydroxy-3,4-dimethyl-2-pyridon;
6-[4-(2,4-Dichlor-benzyl)-phenoxymethyl]-1-hydroxy-3,4-dimethyl-2-pyridon;
6-[4-(Cinnamyloxy)-phenoxymethyl]-1-hydroxy-4-methyl-2-pyridon;
1-Hydroxy-4-methyl-6-[4-(4-trifluormethyl-phenoxy)-phenoxymethyl]-2-pyridon;
1-Hydroxy-4-methyl-6-cyclohexyl-2-pyridon
   oder
1-Hydroxy-4-methyl-6-(2,4,4-trimethylpentyl)-2-pyridon.

Die oben genannten Verbindungen der Formel I können sowohl in freier Form als auch als Salze eingesetzt werden, die Verwendung in freier Form ist bevorzugt.

Die in den Zubereitungen einzusetzenden Wirkstoffe der Verbindung der Formel I können beispielsweise nach Verfahren gemäß US 2 540 218 oder US 4 797 409 hergestellt werden.

Bevorzugt werden immunsupprimierte Patienten behandelt wie Diabetiker, Asthmatiker, Raucher, AIDS-Kranke, Patienten vor und nach Transplantationen, Krebskranke, Patienten, die über lange Zeit mit Antibiotika, Cytostatika oder Corticosteroide behandelt werden, Patienten mit antimykotika-resistenten Pilzen, insbesondere Patienten mit fluconazol-resistenten Pilzen oder ältere Menschen.

Für den erfindungsgemäßen Einsatz der genannten Verbindungen kommen flüssige, halbfeste und feste pharmazeutische Zubereitungen in Betracht, insbesondere Lösungen, Creme-, Salben- und Gelzubereitungen, sowie Lutschtabletten und Vaginalovula.

In den erfindungsgemäßen Zubereitungen wird der Wirkstoff in Mengen eingearbeitet, die üblicherweise zwischen etwa 0,05 und etwa 5 %, vorzugsweise zwischen 0,1 und 1 % liegen.

Mit den erfindungsgemäßen Arzneimitteln läßt sich bei der topischen Behandlung von Candidosen, die durch Hefestämme mit intrinscher und erworbener fluconazolresistenz verursacht werden, eine durchgreifende Heilung erzielen.

### Beispiel 1

Eine erfindungsgemäße Zubereitung weist folgende Zusammensetzung auf:

| | |
|---|---|
| 6-[4-(4-Chlor-phenoxy)-phenoxymethyl]-1-hydroxy-4-methyl-2(1H)-pyridon | 0,50 % |
| Hydroxyethylcellulose | 1,50 % |
| Polyethylenglykol-7 Glycerylcocoat | 5,00 % |
| 1,2-Propylenglykol | 10,00 % |
| Isopropylalkohol | 20,00 % |
| Demineralisiertes Wasser | 63,00 % |

### Beispiel 2

Eine erfindungsgemäße Zubereitung weist folgende Zusammensetzung auf:

| | |
|---|---|
| 6-[4-(4-Chlor-phenoxy)-phenoxymethyl]-1-hydroxy-4-methyl-2(1H)-pyridon | 1,00 % |
| 2-Octyldodecanol | 5,00 % |
| Paraffinöl | 5,00 % |
| Cetylalkohol | 5,00 % |
| Stearylalkohol | 5,00 % |
| Myristylalkohol | 5,00 % |
| Polyoxyethylen-20-sorbitanmonostearat | 3,00 % |
| Sorbitanmonostearat | 2,00 % |
| Demineralisiertes Wasser | 69,00 % |

### Beispiel 3

Eine erfindungsgemäße Zubereitung weist folgende Zusammensetzung auf:

| | |
|---|---|
| 6-Cyclohexyl-1-hydroxy-4-methyl-2(1H)-pyridon | 5 mg |
| Polyethylenglykol 1500 | 1500 mg |
| Polyethylenglykol 4000 | 1000 mg |
| Polyethylenglykol 6000 | 165 mg |
| Natriumhydrogencarbonat | 180 mg |
| Weinsäure | 150 mg |

### Beispiel 4

Eine erfindungsgemäße Zubereitung weist folgende Zusammensetzung auf:

| | |
|---|---|
| 6-[4-(4-Chlor-phenoxy)-phenoxymethyl]-1-hydroxy-4-methyl-2(1H)-pyridon | 10 mg |
| Tylose C 1000 P | 30 mg |
| Polyethylenglykol 6000 | 500 mg |
| Mannit | 305 mg |
| Natriumstearylfumarat | 5 mg |

### Beispiel 5

### Wirksamkeitsprüfung

### Bestimmung der Wirksamkeit von 6-[4-(4-Chlor-phenoxy)-phenoxy-methyl]-1-hydroxy-4-methyl-2(1H)-pyridon (Verbindung 1) gegenüber Fluconazol resistenten Stämmen von Candida albicans

Die Isolierung von Fluconazol resistenten Stämmen von Candida albicans erfolgt bei Patienten, die beispielsweise länger als ein Jahr mit Fluconazol behandelt wurden. Dazu werden den Patienten im Mundbereich Proben entnommen und unverdünnt oder 1:100 verdünnt auf einem RPMI 1640 Agar (Gibco/BRL, Life Technologies GmbH, D-76339 Eggenstein) aufgebracht, der etwa 1,0 µg/ml Fluconazol enthält. Resistente Candida albicans Stämme werden isoliert, weiter auf Agar gereinigt und in Pepton Dextrose Schrägagarröhrchen isoliert aufbewahrt.

Die Bestimmung der Aktivität von Verbindung 1 und Fluconazol erfolgt mit Hilfe der Mikrotiter Verdünnungstechnik in RPMI 1640-Medium. Das Wachstumsmedium RPMI 1640, gepuffert mit 0,165 M Morpholinopropansulfonsäure pH 7,0, wird in 96-well Mikrotiterplatten gefüllt. Die Verbindung 1 und Fluconazol werden seriell um den Faktor 2 verdünnt, so daß Endkonzentrationen von 256 bis 0,002 µg/ml der Verbindung 1 und Fluconazol erhalten werden. Die so vorbereiteten Mikrotiterplatten werden mit den zu prüfenden Candida Stämmen inkubiert. Zu Vergleichszwecken werden die beiden nicht Fluconazol resistenten Stämme Candida albicans ATCC 90028 und 90029 in die Prüfung einbezogen. Die Anfangszellzahl beträgt 1-5 x 10³ Kolonie bildende Einheiten je ml Wachstumsmedium. Die Mikrotiterplatten werden 48 Stunden bei 35 °C inkubiert. Die minimale Hemmkonzentration wird photometrisch bei 510 nm bestimmt. Tabelle 1 zeigt die Ergebnisse:

**Tabelle 1**

| | **Minimale Hemmkonzentration (MHK) (µg/ml)** | |
|---|---|---|
| **Stamm** | **Fluconazol** | **Verbindung 1** |
| Candida albicans ATCC 90028 | 0,5 | 1 |
| Candida albicans ATCC 90029 | 1 | 0,5 |
| Candida albicans 94/3 | 32 | 2 |
| Candida albicans 94/14 | 32 | 2 |
| Candida albicans 94/57 | > 256 | 1 |
| Candida albicans 94/62 | > 256 | 1 |
| Candida albicans 94/90 | > 256 | 1 |
| Candida albicans 94/118 | > 256 | 2 |
| Candida albicans 94/134 | > 256 | 1 |
| Candida albicans 94/138 | > 256 | 1 |
| Candida albicans 94/222 | > 256 | 1 |
| Candida albicans 94/231 | > 256 | 1 |
| Candida albicans B6 | 1 | 1 |
| Candida albicans B70 | > 256 | 1 |
| Candida albicans B75 | 2 | 1 |
| Candida krusei B1 | 32 | 0,5 |
| Candida krusei B4 | 16 | 1 |
| Candida glabrata B12 | 4 | 0,5 |
| Candida glabrata B14 | 2 | 0,5 |
| Candida glabrata B18 | 8 | 0,5 |
| Candida glabrata B21 | 8 | 1 |
| Candida glabrata B35 | 8 | 1 |
| Candida glabrata B37 | 16 | 1 |
| Candida glabrata B38 | 16 | 1 |
| Candida glabrata B39 | 8 | 0,5 |
| Candida glabrata B40 | 16 | 1 |
| Candida glabrata B50 | 16 | 0,5 |
| Candida glabrata B51 | 32 | 0,5 |
| Candida guillermondii B47 | 4 | 1 |

Tabelle 1 zeigt, daß Verbindung 1 - unabhängig von einer bestehenden Fluconazol Resistenz - Candida Stämme in einem sehr engen Konzentrationsbereich am Wachstum hindert.

## Patentansprüche

1. Verwendung der Verbindung der Formel I 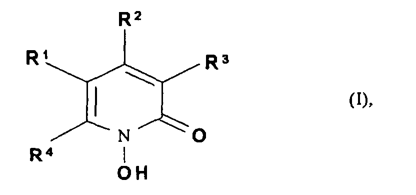 worin
R¹, R² und R³, die gleich oder verschieden sind, Wasserstoffatom oder Alkyl mit 1 - 4 Kohlenstoffatomen bedeuten, und
R⁴ einen gesättigten Kohlenwasserstoffrest mit 6 bis 9 Kohlenstoffatomen oder einen Rest der Formel II bedeutet 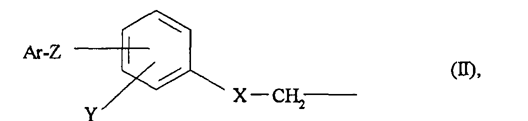 wobei
X S oder O bedeutet,
Y Wasserstoffatom oder bis zu 2 Halogenatome wie Chlor und/oder Brom bedeutet,
Z eine Einfachbindung oder die zweiwertigen Reste O, S, -CR₂-(R = H oder C₁-C₄-Alkyl) oder andere zweiwertige Reste mit 2 bis 10 kettenförmig verknüpften C- und gegebenenfalls O- und/oder S-Atomen, wobei - wenn die Reste 2 oder mehr O- und/oder S-Atome enthalten - letztere durch mindestens 2 C-Atome voneinander getrennt sein müssen und wobei 2 benachbarte C-Atome auch durch eine Doppelbindung miteinander verknüpft sein können und die freien Valenzen der C-Atome durch H und/oder C₁-C₄-Alkylgruppen abgesättigt sind, bedeutet,
Ar ein aromatisches Ringsystem mit bis zu zwei Ringen, das durch bis zu drei Reste aus der Gruppe Fluor, Chlor, Brom, Methoxy, C₁-C₄-Alkyl, Trifluormethyl und Trifluormethoxy substituiert sein kann,
und/oder ein physiologisch verträgliches Salz der Verbindung der Formel I zur Herstellung eines Arzneimittels zur topischen Behandlung von Pilzerkrankungen, die durch fluconazol-resistente Pilze versursacht werden.

2. Verwendung nach Anspruch 1, **dadurch gekennzeichnet, daß** man eine Verbindung der Formel I einsetzt, worin Ar den Phenylring darstellt und R¹ und R³ Wasserstoffatom sind und R² Methyl ist.

3. Verwendung nach Anspruch 1, **dadurch gekennzeichnet, daß** man eine Verbindung der Formel I einsetzt, worin Ar ein bicyclisches System darstellt, das sich vom Biphenyl, Diphenylalkan oder Diphenylether ableitet.

4. Verwendung nach einem oder mehreren der Ansprüche 1 bis 3, **dadurch gekennzeichnet, daß** man eine Verbindung der Formel I einsetzt, worin Z eine Einfachbindung ist oder Z Sauerstoff darstellt.

5. Verwendung nach Anspruch 1, **dadurch gekennzeichnet, daß** man 6-[4-(4-Chlor-phenoxy)-phenoxymethyl]-1-hydroxy-4-methyl-2-pyridon, 1-Hydroxy-4-methyl-6-cyclohexyl-2-pyridon oder 1-Hydroxy-4-methyl-6-(2,4,4-trimethylpentyl)-2-pyridon einsetzt.

6. Verwendung nach einem oder mehreren der Ansprüche 1 bis 5, zur Herstellung eines Arzneimittels zur Behandlung von Pilzerkrankungen, die durch Hefe verursacht werden, die eine intrinsische und/oder erworbene Fluconazolresistenz aufweist.

7. Verwendung nach einem oder mehreren der Ansprüche 1 bis 6 zur Behandlung von Vaginalcandidosen. und/oder ein physiologisch verträgliches Salz der Verbindung der Formel I zur Herstellung eines Arzneimittels zur topischen Behandlung von Pilzerkrankungen, die durch fluconazol-resistente Pilze versursacht werden.

## Claims

1. The use of a compound of the formula I 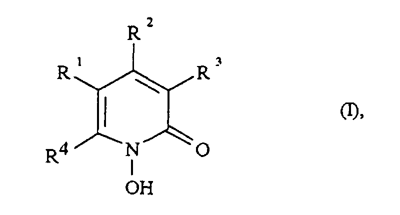
in which R¹, R² and R³, which are identical or different, are hydrogen atom or alkyl with 1-4 carbon atoms, and
R⁴ is a saturated hydrocarbon radical with 6 to 9 carbon atoms or a radical of the formula II 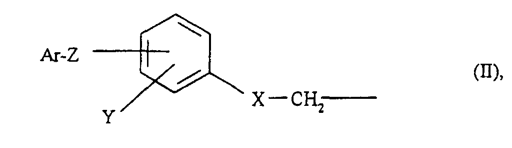 where
X is S or O,
Y is a hydrogen atom or up to 2 halogen atoms such as chlorine and/or bromine,
Z is a single bond or the divalent radicals O, S, -CR₂- (R = H or C₁-C₄-alkyl) or other divalent radicals with 2 to 10 carbon and, where appropriate, oxygen and/or sulfur atoms linked to form a chain, and - when the radicals contain 2 or more oxygen and/or sulfur atoms - the latter must be separated from one another by at least 2 carbon atoms, and where 2 adjacent carbon atoms can also be linked together by a double bond, and the free valencies of the carbon atoms are saturated by H and/or C₁-C₄ alkyl groups,
Ar is an aromatic ring system which has up to two rings and can be substituted by up to three radicals from the group of fluorine, chlorine, bromine, methoxy, C₁-C₄-alkyl, trifluoromethyl and trifluoromethoxy,
and/or a physiologically tolerated salt of the compound of the formula I
for the preparation of a pharmaceutical for the
topical treatment of fungal disorders caused by fluconazole-resistant fungi.

2. The use as claimed in claim 1, wherein a compound of the formula I in which Ar is the phenyl ring, and R¹ and R³ are a hydrogen atom, and R² is methyl, is employed.

3. The use as claimed in claim 1, wherein a compound of the formula I in which Ar is a bicyclic system derived from biphenyl, diphenylalkane or diphenyl ether is employed.

4. The use as claimed in one or more of claims 1 to 3, wherein a compound of the formula I in which Z is a single bond or Z is oxygen is employed.

5. The use as claimed in claim 1, wherein 6-[4-(4-chlorophenoxy)phenoxymethyl]-1-hydroxy-4-methyl-2-pyridone, 1-hydroxy-4-methyl-6-cyclohexyl-2-pyridone or 1-hydroxy-4-methyl-6-(2,4,4-trimethylpentyl)-2-pyridone is employed.

6. The use as claimed in one or more of claims 1 to 5 for the preparation of a pharmaceutical for the treatment of fungal disorders caused by yeast which has an intrinsic and/or acquired fluconazole resistance.

7. The use as claimed in one or more of claims 1 to 6 for the treatment of vaginal candidoses.

## Revendications

1. Utilisation du composé de formule I 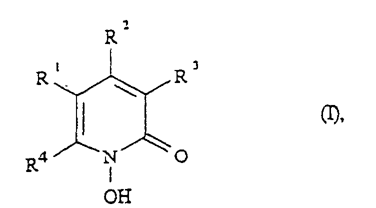 dans laquelle
R¹, R² et R³, qui sont identiques ou différents, représentent chacun un atome d'hydrogène ou un groupe alkyle ayant de 1 à 4 atomes de carbone, et
R⁴ est un radical hydrocarboné saturé ayant de 6 à 9 atomes de carbone ou un radical de formule II 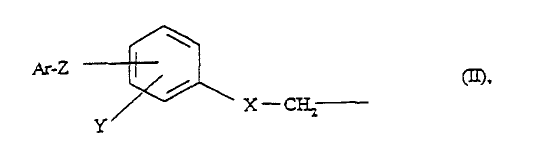 dans laquelle
X est S ou O,
Y est un atome d'hydrogène ou représente jusqu'à 2 atomes d'halogène, tels que des atomes de chlore et/ou de brome,
Z est un liaison simple, ou l'un des radicaux divalents O, S, -CR₂- (R est H ou un groupe alkyle en C₁-C₄), ou un autre radical divalent ayant de 2 à 10 atomes de carbone, et éventuellement d'oxygène et/ou de soufre, liés en chaîne, auquel cas, quand les radicaux contiennent au moins 2 atomes d'oxygène et/ou de soufre, ces derniers doivent être séparés l'un de l'autre par au moins 2 atomes de carbone, et où 2 atomes de carbone voisins peuvent aussi être reliés l'un à l'autre par une double liaison, et les valences libres des atomes de carbone sont saturées par H et/ou des groupes alkyle en C₁-C₄,
Ar est un système cyclique aromatique ayant jusqu'à deux noyaux, qui peut être substitué par jusqu'à trois radicaux choisis dans le groupe constitué par les groupes fluoro, chloro, bromo, méthoxy, alkyle en C₁-C₄, trifluorométhyle et trifluorométhoxy,
et/ou d'un sel physiologiquement compatible du composé de formule I, pour préparer un médicament destiné au traitement topique des maladies fongiques qui sont provoquées par des champignons résistants au fluconazol.

2. Utilisation selon la revendication 1, **caractérisée en ce qu'**on utilise un composé de formule I dans laquelle Ar est le noyau phényle, et R¹ et R³ sont des atomes d'hydrogène et R² est le groupe méthyle.

3. Utilisation selon la revendication 1, **caractérisée en ce qu'**on utilise un composé de formule I dans laquelle Ar est un système bicyclique qui dérive du biphényle, du diphénylalcane ou du diphényléther.

4. Utilisation selon l'une ou plusieurs des revendications 1 à 3, **caractérisée en ce qu'**on utilise un composé de formule I dans laquelle Z est une liaison simple, ou encore Z est un atome d'oxygène.

5. Utilisation selon la revendication 1, **caractérisée en ce qu'**on utilise la 6-[4-(4-chlorophénoxy)-phénoxyméthyl]-1-hydroxy-4-méthyl-2-pyridine, la 1-hydroxy-4-méthyl-6-cyclohexyl-2-pyridone ou la 1-hydroxy-4-méthyl-6-(2,4,4-triméthylpentyl)-2-pyridone.

6. Utilisation selon l'une ou plusieurs des revendications 1 à 5, pour préparer un médicament destiné au traitement de maladies fongiques qui sont provoquées par une levure présentant une résistance intrinsèque et/ou acquise au fluconazol.

7. Utilisation selon l'une ou plusieurs des revendications 1 à 6 pour le traitement des candidoses vaginales.
